# EUROPEAN PATENT SPECIFICATION

(11) **EP 3 137 622 B1**
(45) Date of publication and mention of the grant of the patent: **24.11.2021**
(21) Application number: 15785560.2
(22) Date of filing: 28.04.2015
(51) Int. Cl.: A61K 31/4985, A61K 31/00, A61P 11/00, A61P 25/28

(54) **METHODS OF REDUCING DECLINE IN VITAL CAPACITY**
VERFAHREN ZUR REDUZIERUNG DES RÜCKGANGS DER VITALKAPAZITÄT
MÉTHODES DE RÉDUCTION DU DÉCLIN DE LA CAPACITÉ VITALE

(30) Priority: 29.04.2014 US 201461985799 P; 02.06.2014 US 201462006337 P; 09.07.2014 US 201462022407 P; 19.12.2014 US 201462094542 P
(43) Date of publication of application: 08.03.2017
(73) Proprietor: Cytokinetics, Inc., South San Francisco, CA 94080 (US)
(72) Inventor: SHEFNER, Jeremy, M., Paradise Valley, AZ 85253 (US); WOLFF, Andrew, A., South San Francisco, CA 94080 (US); MALIK, Fady, South San Francisco, California 94080 (US); ANDREWS, Jinsy, A., South San Francisco, California 94080 (US)
(74) Representative: Carpmaels & Ransford LLP
(86) International application number: PCT/US2015/027897
(87) International publication number: WO 2015/168064

(56) References cited:
- WO-A1-2013/151938
- WO-A2-2013/010015
- WO-A2-2013/155262
- US-B2- 8 227 603
- SHEFNER JEREMY ET AL: "Safety, tolerability and pharmacodynamics of a skeletal muscle activator in amyotrophic lateral sclerosis", AMYOTROPHIC LATERAL SCLEROSIS : OFFICIAL PUBLICATION OF THE WORLD FEDERATION OF NEUROLOGY RESEARCH GROUP ON MOTOR NEURON DISEASES 2009 OCT,, vol. 13, no. 5, 1 January 2012 (2012-01-01), pages 430-438, XP009185356, ISSN: 1471-180X, DOI: 10.3109/17482968.2012.684214DOI:10.3109/17 482968.2012.684214
- SHEFNER JEREMY ET AL: "A Phase 2, Single Dose Study of CK-2017357, a Skeletal Muscle Troponin Activator in Patients with ALS", NEURO, LIPPINCOTT WILLIAMS & WILKINS, PHILADELPHIA, US, vol. 76, no. 9, suppl. 4, 1 March 2011 (2011-03-01), page A666, XP009185361, ISSN: 0028-3878
- None

## Description

Vital capacity is the maximum amount of air one can expel from the lungs after a maximum inhalation. Vital capacity is a measure of underlying lung disease (e.g., asthma, pulmonary fibrosis, cystic fibrosis, COPD) and neurodegenerative disease (e.g., amyotrophic lateral sclerosis (ALS), myasthenia gravis, spinal muscle atrophy (SMA), muscular dystrophy). Vital capacity can be measured by spirometry, which is a common pulmonary function test measuring lung function, specifically the amount and/or speed of air that can be inhaled and exhaled. Indications for spirometry include measuring the effect of disease on pulmonary function and monitoring the course of diseases that affect respiratory function. According to a task force established by the American Thoracic Society and the European Respiratory Society (the ATS/ERS Task Force), spirometry is invaluable as a screening test of general respiratory health. One of the most important aspects of spirometry includes vital capacity (Miller, M. R., et al. (2005), Eur.Respir.J. 26: 319―338).

Vital capacity can be measured either by a forced exhalation maneuver, yielding forced vital capacity (FVC), or a slow exhalation maneuver, yielding slow vital capacity (SVC, also referred to in the literature as VC or expiratory vital capacity). SVC measures the volume of air expired non-forcefully in one breath and is a more accurate measure of vital capacity than FVC in the setting of advancing disease.

Respiratory failure is the primary cause of death in ALS, therefore measurements of respiratory muscle function are very important in clinically assessing patients with ALS. ALS patients can develop poor or uncoordinated glottal closure which can cause falsely low or variable FVC measurements (Brinkmann, J., et al. (1997), J.Neurol.Sci. 147: 97-111). Also, when patients with ALS develop weakness of the face and mouth or have upper motor neuron disease impairing rapid coordinated movements, FVC becomes technically difficult to measure (Paillisse, C., et al. (2005), Amyotrophic lateral sclerosis and other motor neuron disorders: Official publication of the World Federation of Neurology, Research Group on Motor Neuron Diseases 6(1): 37-44; Sanjak, M., F. Salachas, et al. (2010), Amyotroph.Lateral.Scler. 4: 383-388). Because of these considerations, SVC has emerged as a preferred method of measuring vital capacity in ALS clinical trials.

ALS is a disease of the nerve cells in the brain and spinal cord that control voluntary muscle movement. In ALS, progressive death of motor neurons leads to denervation of skeletal muscles. Surviving motor units attempt to compensate for dying ones by innervating more muscle fibers (termed sprouting), but are only partially successful (Kiernan MC, et al. (2011), Lancet 377(9769): 942-955). Over time, progressive denervation and its consequent skeletal muscle atrophy lead to weakness, fatigue, and eventually complete paralysis and death, primarily from respiratory complications.

Rilutek® (riluzole, Sanofi-Aventis U.S. LLC) is the only approved medication for the treatment of ALS, prolonging survival or time to tracheostomy by approximately 2-3 months versus placebo (Lacomblez, L., et al. (1996), Lancet 347(9013): 1425-1431). Riluzole, however, does not improve measurable parameters of neuromuscular or pulmonary function. The mechanism by which riluzole prolongs life in ALS has not been confirmed but it is thought to inhibit glutamate release, inactivate voltage-dependent sodium channels, and interfere with intracellular events that follow transmitter binding at excitatory amino acid receptors.

To date, there are no available treatments that can improve skeletal muscle function (and thereby, also respiratory function) in ALS, which has been identified as a potential therapeutic target in ALS (Shefner, J. M. (2009), Exp.Neurol. 219(2): 373-375).

Muscle contraction is driven by the cyclical interaction of myosin thick filaments and actin/troponin/tropomyosin-containing thin filaments. The motor protein myosin hydrolyzes adenosine triphosphate in a cycle that governs its interaction with actin filaments, converting the chemical energy of the phosphate bond into mechanical force. Actin is a filamentous polymer and is the substrate upon which myosin pulls during force generation. Bound to actin are regulatory proteins, the troponin complex and tropomyosin, which make the actin-myosin interaction dependent on changes in intracellular calcium levels. Calcium binding to the troponin complex induces conformational changes that regulate the accessibility of myosin binding sites along the actin filaments that, in the absence of calcium, are blocked by tropomyosin. The troponin complex consists of three components; troponin C (TnC), which binds calcium, troponin T, which acts as a structural linker between actin and tropomyosin, and troponin I, which tethers the troponin complex and tropomyosin to actin and also initiates the movement of tropomyosin upon calcium binding to TnC. Calcium binding to TnC causes a conformational change in the troponin complex that leads to displacement of tropomyosin on the actin filament, exposing myosin binding sites and allowing force development to proceed.

There are three classes of striated muscle: slow skeletal (type I), fast skeletal (type II), and cardiac. Each muscle type has its own unique combination of troponin isoforms that form the troponin complex. Although all troponin isoforms are highly conserved across species (for example, fast skeletal muscle TnC shares 98% amino acid identity between mouse and humans, justifying the use of non-human species for preparation of screening and test materials), at the amino acid level, homology between fast skeletal muscle and cardiac muscle troponin elements is substantially lower (42-64% amino acid identity). This enables a means to generate selective troponin activators.

Selective skeletal muscle troponin activators have been developed based on the hypothesis that amplifying the response of the sarcomere, the fundamental contractile unit in skeletal muscle, to inadequate motor neuron input would improve muscle force generation and physical function in individuals with neuromuscular diseases, such as ALS. One way to amplify the sarcomere response is to increase the calcium sensitivity of the troponin-tropomyosin regulatory complex, which is the calcium sensor within the sarcomere that regulates the actin-myosin force-generating interaction.

Shefner, Jeremy et. al., Amyotrophic Lateral Sclerosis, 2012, vol. 13, no. 5, pages 430-438 discloses a study "designed to evaluate the safety and tolerability of single doses of CK-2017357, an orally bioavailable fast skeletal muscle troponin activator" (see abstract).

WO 2013/010015 A2 discloses "[a] method for treating ALS in a subject, comprising administering to the subject a therapeutically effective amount of riluzole and a therapeutically effective amount of CK-2017357" (see abstract).

Jeremy Shefner et. al., Neuro., Lippincott Williams & Wilkins, 2011, vol. 76, no. 9, suppl. 4, 1 page A666, ISSN: 0028-3878 discloses "A Phase 2, Single Dose Study of CK-2017357, a Skeletal Muscle Troponin Activator in Patients with ALS" (see title).

WO 2013/151938 A1 discloses "[c]ompositions and methods for improving diaphragm function in a patient" (see abstract).

The invention provides a skeletal muscle troponin activator for use in a method of reducing decline in slow vital capacity in a subject, the method comprising administering to the subject a therapeutically effective amount of the skeletal muscle troponin activator, wherein the skeletal muscle troponin activator is 1-(2-(((3-fluoro-1-(3-fluoropyridin-2-yl)cyclobutyl)methyl)amino)pyrimidin-5-yl)-1H-pyrrole-3-carboxamide, or a pharmaceutically acceptable salt thereof. The invention also provides a skeletal muscle troponin activator for use in a method of reducing progressive respiratory decline in a subject with amyotrophic lateral sclerosis (ALS), the method comprising administering to the subject a therapeutically effective amount of the skeletal muscle troponin activator, wherein the skeletal muscle troponin activator is 1-(2-(((3-fluoro-1-(3-fluoropyridin-2-yl)cyclobutyl)methyl)amino)pyrimidin-5-yl)-1H-pyrrole-3-carboxamide, or a pharmaceutically acceptable salt thereof, wherein the decline of the subject's slow vital capacity (SVC) is reduced.

In some embodiments, the subject receiving such administration suffers from a lung disease (e.g., asthma, pulmonary fibrosis, cystic fibrosis, or COPD) or a neurodegenerative disease (e.g., amyotrophic lateral sclerosis (ALS), myasthenia gravis, spinal muscle atrophy (SMA), or muscular dystrophy). In some embodiments, the subject suffers from ALS. In some embodiments, a fast skeletal muscle troponin activator is administered to a subject suffering from ALS to reduce decline in slow vital capacity. In some embodiments, a fast skeletal muscle troponin activator is administered to a subject suffering from ALS to reduce progressive respiratory decline, as measured by SVC.

### Brief Description of the Figures

**FIG. 1** depicts the design of the BENEFIT-ALS clinical trial.
**FIG. 2** depicts the doses of placebo or tirasemtiv that patients received during each week of the double-blind phase of the study.
**FIG. 3** depicts the difference in the slope of change from baseline between placebo and tirasemtiv in ALSFRS-R, % predicted SVC, MVV, SNIP, grip fatigue and muscle mega-score.
**FIG. 4** depicts the changes from baseline in SVC and the slope of the changes from baseline for placebo and tirasemtiv.
**FIG. 5** depicts subgroup analyses of change from baseline between placebo and tirasemtiv in SVC.
**FIG. 6** depicts the difference in weight loss between placebo and tirasemtiv in patients with and without a gastrointestinal adverse event.
**FIG. 7** depicts the impact of weight loss on the effect of tirasemtiv on ALSFRS-R versus placebo.

The compounds described herein include crystalline and amorphous forms of those compounds, including, for example, polymorphs, pseudopolymorphs, unsolvated polymorphs (including anhydrates), conformational polymorphs of the compounds, as well as mixtures thereof. In certain embodiments, the compounds described herein are in the form of pharmaceutically acceptable salts. In certain embodiments, the compounds described herein are in the form of pharmaceutically acceptable solvates (including, but not limited to, hydrates). In certain embodiments, the compounds described herein are in the form of pharmaceutically acceptable solvates of pharmaceutically acceptable salts.

The term "pharmaceutically acceptable salt" refers to salts that retain the biological effectiveness and properties of the compounds described herein and, which are not biologically or otherwise undesirable. In many cases, the compounds described herein are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. In some embodiments, the pharmaceutically acceptable base addition salt is chosen from ammonium, potassium, sodium, calcium, and magnesium salts.

The term "solvate" refers to a compound in physical association with a pharmaceutically acceptable solvent. A compound molecule may be associated with any number of solvent molecules. For example, the ratio of compound to solvent molecules may be 1:1 (e.g., a hydrate), 2:1 (e.g., a hemihydrate), 1:2 (e.g., a dihydrate), or any other ratio that leads to a stable solvate. It will be understood that "a compound of Formula X" encompasses the compound of Formula X, solvates of those compounds, and mixtures thereof.

The compounds described herein can be enriched isotopic forms, e.g., enriched in the content of ²H, ³H, ¹¹C, ¹³C and/or ¹⁴C. In some embodiments, the compound contains at least one deuterium atom. Such deuterated forms can be made, for example, by the procedure described in U.S. Patent Nos. 5,846,514 and 6,334,997. Such deuterated compounds may improve the efficacy and increase the duration of action of compounds described herein. Deuterium substituted compounds can be synthesized using various methods, such as those described in: Dean, D., Recent Advances in the Synthesis and Applications of Radiolabeled Compounds for Drug Discovery and Development, Curr. Pharm. Des., 2000; 6(10); Kabalka, G. et al., The Synthesis of Radiolabeled Compounds via Organometallic Intermediates, Tetrahedron, 1989, 45(21), 6601-21; and Evans, E., Synthesis of radiolabeled compounds, J. Radioanal. Chem., 1981, 64(1-2), 9-32.

As used herein, "skeletal muscle" includes skeletal muscle tissue as well as components thereof, such as skeletal muscle fibers, the myofibrils comprising the skeletal muscle fibers, the skeletal sarcomere which comprises the myofibrils, and the various components of the skeletal sarcomere described herein, including skeletal myosin, actin, tropomyosin, troponin C, troponin I, troponin T and fragments and isoforms thereof. In some embodiments, "skeletal muscle" includes fast skeletal muscle tissue as well as components thereof, such as fast skeletal muscle fibers, the myofibrils comprising the fast skeletal muscle fibers, the fast skeletal sarcomere which comprises the myofibrils, and the various components of the fast skeletal sarcomere described herein, including fast skeletal myosin, actin, tropomyosin, troponin C, troponin I, troponin T and fragments and isoforms thereof. Skeletal muscle does not include cardiac muscle or a combination of sarcomeric components that occurs in such combination in its entirety in cardiac muscle.

As used herein, "selective binding" or "selectively binding" refers to preferential binding to a target protein in one type of muscle or muscle fiber as opposed to other types. For example, a compound selectively binds to fast skeletal troponin C if the compound preferentially binds troponin C in the troponin complex of a fast skeletal muscle fiber or sarcomere in comparison with troponin C in the troponin complex of a slow muscle fiber or sarcomere or with troponin C in the troponin complex of a cardiac sarcomere.

The terms "patient" and "subject" refer to an animal, such as a mammal, bird or fish. In some embodiments, the patient or subject is a mammal. Mammals include, for example, mice, rats, dogs, cats, pigs, sheep, horses, cows and humans. In some embodiments, the patient or subject is a human, for example a human that has been or will be the object of treatment, observation or experiment. The compounds, compositions and methods described herein can be useful in both human therapy and veterinary applications.

"Treatment" or "treating" means any treatment of a disease in a subject, including one or more of: preventing the disease, i.e., causing the clinical symptoms of the disease not to develop; inhibiting the disease; slowing or arresting the development of clinical symptoms; and/or relieving the disease, i.e., causing the regression of clinical symptoms.

As used herein, the term "therapeutic" refers to a beneficial or desirable consequence of a medical treatment. In certain instances, "therapeutic" refers to the ability to modulate the contractility of fast skeletal muscle. As used herein, "modulation" (and related terms, such as "modulate", "modulated", "modulating") refers to a change in function or efficiency of one or more components of the fast skeletal muscle sarcomere, including myosin, actin, tropomyosin, troponin C, troponin I, and troponin T from fast skeletal muscle, including fragments and isoforms thereof, as a direct or indirect response to the presence of a compound described herein, relative to the activity of the fast skeletal sarcomere in the absence of the compound. The change may be an increase in activity (potentiation) or a decrease in activity (inhibition), and may be due to the direct interaction of the compound with the sarcomere, or due to the interaction of the compound with one or more other factors that in turn affect the sarcomere or one or more of its components. In some embodiments, modulation is a potentiation of function or efficiency of one or more components of the fast skeletal muscle sarcomere, including myosin, actin, tropomyosin, troponin C, troponin I, and troponin T from fast skeletal muscle, including fragments and isoforms thereof. Modulation may be mediated by any mechanism and at any physiological level, for example, through sensitization of the fast skeletal sarcomere to contraction at lower Ca²⁺ concentrations.

The term "therapeutically effective amount" or "effective amount" refers to that amount of a compound described herein that is sufficient to effect treatment, as defined below, when administered to a subject in need of such treatment. The therapeutically effective amount will vary depending upon the subject and disease condition being treated, the weight and age of the subject, the severity of the disease condition, the particular compound selected from the disclosed formulas, the dosing regimen to be followed, timing of administration, the manner of administration and the like, all of which can readily be determined by one of ordinary skill in the art.

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

In some embodiments, the subject suffers from a disease or condition that affects pulmonary function. Non-limiting examples of such diseases and conditions include multiple sclerosis, stroke, Arnold-Chiari malformation, quadriplegia, amyotrophic lateral sclerosis (ALS), poliomyelitis, spinal muscular atrophy (SMA), syringomyelia, Guillain-Barre syndrome, tumor compression, neuralgic neuropathy, critical-illness polyneuropathy, chronic inflammatory demyelinating polyneuropathy, Charcot-Marie-Tooth disease, idiopathic, chronic obstructive pulmonary disease (COPD), asthma, myasthenia gravis, Lambert-Eaton syndrome, botulism, organophosphate exposure, drug use, muscular dystrophies (including Duchenne muscular dystrophy, Becker muscular dystrophy, limb-girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy), myositis (infectious, inflammatory, metabolic), acid maltase deficiency, glucocorticoids, disuse atrophy, pulmonary fibrosis, cystic fibrosis, sleep-disordered breathing, ventilator-induced diaphragmatic weakness or atrophy, steroid-induced diaphragmatic atrophy, hemidiaphragm paralysis, fetal hydrops, pleural effusion, or phrenic nerve dysfunction. Thus, provided is 1-(2-(((3-fluoro-1-(3-fluoropyridin-2-yl)cyclobutyl)methyl)amino)pyrimidin-5-yl)-1H-pyrrole-3-carboxamide for use in reducing the decline in slow vital capacity in a subject suffering from any one or more of these diseases or conditions.

A primary cause of morbidity and mortality in patients with ALS is due to respiratory failure. By improving respiratory function by administration of a fast skeletal troponin activator, ALS patient quality of life may be improved. In some embodiments, the decline in slow vital capacity is reduced in a subject with ALS by administering to the subject a therapeutically effective amount of the skeletal muscle troponin activator. The reduction in decline of slow vital capacity is relative to, for example, one or more comparable subjects, or an average of a group of comparable subjects, who are not receiving the skeletal muscle troponin activator. In some embodiments, the decline of percent predicted vital capacity is reduced by 5%, 10%, 15%, 20%, 25%, 30%, 35%, 40%, 45%, 50%, 60%, 70% 80% or 90% compared to one or more comparable subjects, or an average of a group of comparable subjects, who are not receiving a skeletal muscle troponin activator. The reduction in respiratory decline as measured by percent predicted SVC is relative to, for example, one or more comparable subjects, or an average of a group of comparable subjects, who are not receiving the skeletal muscle troponin activator. In some embodiments, the respiratory decline as measured by percent predicted SVC is reduced by 5%, 10%, 15%, 20%, 25%, 30%, 35%, 40%, 45%, 50%, 60%, 70% 80% or 90% compared to one or more comparable subjects, or an average of a group of comparable subjects, who are not receiving the skeletal muscle troponin activator.

In some embodiments, the patient has a baseline SVC lower than about 80%, or alternatively lower than about 75%, 70%, 65%, 60%, 55%, 50%, 45%, 40%, 35%, 30%, 25% or 20% of predicted of healthy individual in similar conditions. In some embodiments, the patient has a baseline forced vital capacity (FVC) lower than about 80%, or alternatively lower than about 75%, 70%, 65%, 60%, 55%, 50%, 45%, 40%, 35%, 30%, 25% or 20% of predicted of healthy individual in similar conditions. In some embodiments, the patient shows evidence of increased work of breathing indicative of reduced pulmonary function, e.g., significant tachypnea, intercostal retractions, or other physical signs of respiratory distress.

Two additional measures of pulmonary function are maximal static inspiratory pressure and sniff nasal inspiratory pressure. In some embodiments, the patient has a maximal static inspiratory pressure or sniff nasal inspiratory pressure that is lower than about 80%, or alternatively lower than about 75%, 70%, 65%, 60%, 55%, 50%, 45%, 40%, 35%, 30%, 25% or 20% of predicted of healthy individual in similar conditions.

Direct measures of pulmonary function include invasive methods such as transdiaphragmatic pressure [Pdi] or noninvasive means such as ultrasonography. Here, a sniff Pdi or Pdi max greater than 80 cm of water in men and greater than 70 cm of water in women rules out clinically significant diaphragmatic weakness. A twitch Pdi greater than 10 cm of water with unilateral phrenic-nerve stimulation or greater than 20 cm of water with bilateral phrenic-nerve stimulation also rules out clinically significant weakness. In some embodiments, the patient is a male patient having a sniff Pdi or Pdi max lower than about 80 cm of water, or alternatively lower than about 75 cm, 70 cm, 65 cm, 60 cm, 55 cm, 50 cm, 45 cm, 40 cm, 35 cm, 30 cm, or 25 cm of water. In some embodiments, the patient is a female patient having a sniff Pdi or Pdi max lower than about 70 cm of water, or alternatively lower than about 65 cm, 60 cm, 55 cm, 50 cm, 45 cm, 40 cm, 35 cm, 30 cm, 25 cm, or 20 cm of water. In some embodiments, the patient has a twitch Pdi lower than about 10 cm, or alternatively lower than about 9 cm, 8 cm, 7 cm, 6 cm, 5 cm, 4 cm, 3 cm, 2 cm or 1 cm of water with unilateral phrenic-nerve stimulation. In some embodiments, the patient has a twitch Pdi lower than about 20 cm, or alternatively lower than about 19 cm, 18 cm, 17 cm, 16 cm, 15 cm, 14 cm, 13 cm, 12 cm, 11 cm, 10 cm, 9 cm, 8 cm, 7 cm, 6 cm, 5 cm, 4 cm, 3 cm, 2 cm or 1 cm of water with bilateral phrenic-nerve stimulation.

In some embodiments, the skeletal muscle troponin activator is for use in a method of reducing the decline in slow vital capacity in a subject by and/or reducing progressive respiratory decline in a subject, as measured by slow vital capacity (SVC), described herein wherein the method further comprises administering to the patient a second therapeutic agent. In some embodiments, the second therapeutic agents may be an ALS treatment, such as riluzole. In some embodiments, when riluzole is administered to the subject in addition to the skeletal muscle troponin activator, the dose of riluzole is lowered (e.g., lowered by 75% or 50% or 25%). When a second therapeutic agent is employed in combination with the compounds and compositions described herein, the second therapeutic agent may be used, for example, in those amounts indicated in the Physicians' Desk Reference (PDR) or as otherwise determined by one of ordinary skill in the art.

The skeletal muscle troponin activators described herein are administered at a therapeutically effective dosage, e.g., a dosage sufficient to provide treatment for the disease states previously described. While human dosage levels have yet to be optimized for the compounds described herein, generally, a daily dose ranges from about 0.05 to 100 mg/kg of body weight; in certain embodiments, from about 0.10 to 10.0 mg/kg of body weight; and in certain embodiments, from about 0.15 to 1.0 mg/kg of body weight. Thus, for administration to a 70 kg person, in certain embodiments, the dosage range would be about from 3.5 to 7000 mg per day; in certain embodiments, about from 7.0 to 700.0 mg per day, and in certain embodiments, about from 10.0 to 100.0 mg per day. The amount of the skeletal muscle troponin activator administered will, of course, be dependent on the subject and disease state being treated, the severity of the affliction, the manner and schedule of administration and the judgment of the prescribing physician. For example, a dose range for administration may be from about 100 to 1000 mg per day. In some embodiments, the skeletal muscle troponin activator is administered once a day. In some embodiments, the skeletal muscle troponin activator is administered twice a day. In some embodiments, the skeletal muscle troponin activator is administered in two equal does per day. In some embodiments, the skeletal muscle troponin activators is administered in two unequal doses per day (e.g., a larger first dose than the second dose, or a larger second dose than the first dose). In some embodiments, a first daily dose of a skeletal muscle troponin activator is administered for a first time period, then a second daily dose of a skeletal muscle troponin activator is administered after the first time period. For example, the first time period may be 1 day, 2 days, 3 days, 4 days, 5 days, 6 days, 1 week, 2 weeks, 3 weeks, 4 weeks, 5 weeks, 6 weeks, 7 weeks or 8 weeks. The first daily dose may be smaller than the second daily dose. Alternatively, the first daily dose may be larger than the second daily dose.

Administration of the skeletal muscle troponin activators described herein can be via any of the accepted modes of administration including, but not limited to, orally, sublingually, subcutaneously, intravenously, intranasally, topically, transdermally, intraperitoneally, intramuscularly, intrapulmonarilly, vaginally, rectally, or intraocularly. In some embodiments, the skeletal muscle troponin activator is administered orally.

Pharmaceutically acceptable compositions include solid, semi-solid, liquid and aerosol dosage forms, such as, e.g., tablets, capsules, powders, liquids, suspensions, suppositories, aerosols or the like. The skeletal muscle troponin activators can also be administered in sustained or controlled release dosage forms, including depot injections, osmotic pumps, pills, transdermal (including electrotransport) patches, and the like, for prolonged and/or timed, pulsed administration at a predetermined rate. In certain embodiments, the compositions are provided in unit dosage forms suitable for single administration of a precise dose.

The skeletal muscle troponin activators described herein can be administered either alone or more typically in combination with a conventional pharmaceutical carrier, excipient or the like (e.g., mannitol, lactose, starch, magnesium stearate, sodium saccharine, talcum, cellulose, sodium crosscarmellose, glucose, gelatin, sucrose, magnesium carbonate, and the like). If desired, the pharmaceutical composition can also contain minor amounts of nontoxic auxiliary substances such as wetting agents, emulsifying agents, solubilizing agents, pH buffering agents and the like (e.g., sodium acetate, sodium citrate, cyclodextrine derivatives, sorbitan monolaurate, triethanolamine acetate, triethanolamine oleate, and the like). Generally, depending on the intended mode of administration, the pharmaceutical composition will contain about 0.005% to 95%; in certain embodiments, about 0.5% to 50% by weight of a skeletal muscle troponin activator. Actual methods of preparing such dosage forms are known, or will be apparent, to those skilled in this art; for example, see Remington's Pharmaceutical Sciences, Mack Publishing Company, Easton, Pennsylvania.

In certain embodiments, the compositions will take the form of a pill or tablet and thus the composition will contain, along with the active ingredient, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (e.g., in propylene carbonate, vegetable oils or triglycerides) is encapsulated in a gelatin capsule.

Liquid pharmaceutically administrable compositions can, for example, be prepared by dissolving, dispersing, etc. a skeletal muscle troponin activator and optional pharmaceutical adjuvants in a carrier (e.g., water, saline, aqueous dextrose, glycerol, glycols, ethanol or the like) to form a solution or suspension. Injectables can be prepared in conventional forms, either as liquid solutions or suspensions, as emulsions, or in solid forms suitable for dissolution or suspension in liquid prior to injection. The percentage of the skeletal muscle troponin activator contained in such parenteral compositions is highly dependent on the specific nature thereof, as well as the activity of the chemical entities and the needs of the subject. However, percentages of active ingredient of 0.01% to 10% in solution are employable, and will be higher if the composition is a solid which will be subsequently diluted to the above percentages. In certain embodiments, the composition will comprise from about 0.2 to 2% of the active agent in solution.

Pharmaceutical compositions of the skeletal muscle troponin activators described herein may also be administered to the respiratory tract as an aerosol or solution for a nebulizer, or as a micro fine powder for insufflation, alone or in combination with an inert carrier such as lactose. In such a case, the particles of the pharmaceutical composition have diameters of less than 50 microns, in certain embodiments, less than 10 microns.

Skeletal muscle troponin activators suitable for the methods described herein can be selected from compounds disclosed in U.S. Patent Nos. 7,598,248, 7,851,484, 7,956,056, 7,989,469, 7,998,976, 8,686,007, 8,759,380, 8,962,632, and 8,969,346, and U.S. Patent Publication Nos. 2010/0173930 and 2013/0150368.

In some embodiments, a skeletal muscle troponin activator (not that of the claims) is tirasemtiv, i.e., 6-ethynyl-1-(pentan-3-yl)-1H-imidazo[4,5-b]pyrazin-2(3H)one (CA Index name: 2H-Imidazo[4,5-b]pyrazin-2-one, 1-(1-ethylpropyl)-6-ethynyl-1,3-dihydro; also known as CK-2017357), or a pharmaceutically acceptable salt thereof. The structure of tirasemtiv is as follows:

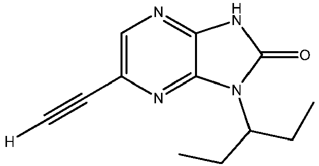

Tirasemtiv and methods for preparing the compound are disclosed in U.S. Patent No. 7,598,248. Tirasemtiv is a highly selective activator of the fast skeletal muscle troponin complex and was developed as a means to increase muscle strength by amplifying the response of muscle when neuromuscular input is diminished secondary to a neuromuscular disease. Tirasemtiv slows the rate of calcium release from fast skeletal TnC, increasing its affinity for calcium and thus sensitizes muscle to calcium. As a consequence, the force-calcium relationship of muscle fibers shifts leftwards and muscle force increases relative to control. Tirasemtiv is selective for fast skeletal muscle troponin with little effect on slow skeletal muscle troponin and no effect on cardiac muscle troponin. By sensitizing the fast skeletal troponin complex to calcium, tirasemtiv amplifies the response of muscle to submaximal nerve stimulation. In preclinical models of limited neuromuscular input, tirasemtiv increased the force of muscle contraction at submaximal nerve stimulation frequencies and increased grip strength (Malik, F., et al. (2012), The fast skeletal troponin activator, CK-2017357, increases muscle function and survival in SOD1 (G93A) mice; a model of ALS, American Academy of Neurology 64th Annual Meeting in New Orleans, LA, New Orleans, LA; Russell, A., et al. (2012), Nat Med 18(3): 452-455). In other model systems, tirasemtiv increased muscle power and decreased fatigability of muscle (Hinken, A., et al. (2010), The fast skeletal troponin activator, CK-2017357, reduces muscle fatigue in an in situ model of vascular Insufficiency, Society for Vascular_Medicine's 2010 Annual Meeting: 21st Annual Scientific Sessions, Cleveland, OH; Kennedy, A., et al. (2012), The fast skeletal troponin activator, CK-2017357, improves resistance to fatigue in healthy, conscious rats, 2012 Experimental Biology Annual Conference, San Diego, CA). The time course of the pharmacological effects of the drug is rapid and appears to parallel its pharmacokinetic profile (Russell, A., et al. (2012), Nat Med 18(3): 452-455; Hansen, R., et al. (2014), Muscle Nerve 50(6) 925-931).

Tirasemtiv has been studied in several human clinical trials for the treatment of conditions associated with skeletal muscle function. Because tirasemtiv has been demonstrated both to amplify skeletal muscle force production in response to diminished neuronal input and to delay the onset and reduce the magnitude of skeletal muscle fatigue during repeated or sustained efforts, it may be useful in the treatment of patients with ALS.

In some embodiments, a skeletal muscle troponin activator (not that of the claims) is tirasemtiv and the daily dose of tirasemtiv is 100 to 1000 mg per day. In some embodiments, the daily dose of tirasemtiv is 125 to 500 mg per day. In some embodiments, the daily dose of tirasemtiv is 250 to 1000 mg per day. In some embodiments, the daily dose of tirasemtiv is 250 to 500 mg per day. In some embodiments, the daily dose of tirasemtiv is 250 to 375 mg per day. In some embodiments, the daily dose of tirasemtiv is 375 to 1000 mg per day. In some embodiments, the daily dose of tirasemtiv is 375 to 500 mg per day. In some embodiments, the daily dose of tirasemtiv is 250 mg per day. In some embodiments, the daily dose of tirasemtiv is 375 mg per day. In some embodiments, the daily dose of tirasemtiv is 500 mg per day. In some embodiments, the tirasemtiv is administered once a day. In some embodiments, the tirasemtiv is administered twice a day (i.e., the daily dose of tirasemtiv is divided into two separate doses administered at two different times in the day). In some embodiments, the daily dose of tirasemtiv is administered in two equal does per day (e.g., two equal doses of 125 mg, or two equal doses of 250 mg). In some embodiments, the daily dose of tirasemtiv is administered in two unequal doses per day (e.g., a larger first dose than the second dose (e.g., 250 mg first then 125 mg second; or 500 mg first then 125 mg second; or 500 mg first then 250 mg second), or a larger second dose than the first dose (e.g., 125 mg first then 250 mg second; or 125 mg first then 500 mg second; or 250 mg first then 500 mg second)). In some embodiments, a first daily dose of tirasemtiv is administered for a first time period, then a second daily dose of tirasemtiv is administered after the first time period. For example, the first time period may be 1 day, 2 days, 3 days, 4 days, 5 days, 6 days, 1 week, 2 weeks, 3 weeks, 4 weeks, 5 weeks, 6 weeks, 7 weeks or 8 weeks. The first daily dose may be smaller than the second daily dose (e.g., 250 mg first daily dose, 375 mg second daily dose; or 250 mg first daily dose, 500 mg second daily dose). Alternatively, the first daily dose may be larger than the second daily dose (e.g., 375 mg first daily dose, 250 mg second daily dose; or 500 mg first daily dose, 375 mg second daily dose; or 500 mg first daily dose, 250 mg second daily dose). In some embodiments, tirasemtiv is administered in an amount sufficient to maintain a mean plasma concentration of at least about 5 µg/ml for 24 hours, or about 10 µg/ml for 24 hours, or about 12 µg/ml for 24 hours, or about 14 µg/ml for 24 hours, or about 16 µg/ml for 24 hours, or about 20 µg/ml for 24 hours.

The skeletal muscle troponin activator is 1-(2-(((3-fluoro-1-(3-fluoropyridin-2-yl)cyclobutyl)methyl)amino)pyrimidin-5-yl)-1H-pyrrole-3-carboxamide or a pharmaceutically acceptable salt thereof, as disclosed in U.S. Patent No. 8,962,632.

The following examples serve to more fully describe the disclosed compounds the methods. It is understood that these examples in no way serve to limit the true scope of this invention, but rather are presented for illustrative purposes.

### Example 1: BENEFIT-ALS Clinical Trial

BENEFIT-ALS was a multi-national, randomized, stratified, double-blind, placebo-controlled, parallel-group study of tirasemtiv in patients with ALS. The study consisted of a screening period, an open-label/lead-in period, and a double-blind treatment period. Following screening, eligible patients received open-label tirasemtiv, 125 mg BID, for 7 days. Patients who tolerated the 7-day lead-in period were randomized in a 1:1 ratio to tirasemtiv or placebo. Over the first 3 weeks of double-blind treatment, patients randomized to tirasemtiv had their TDD up-titrated as follows: 250 mg for 7 days, then 375 mg for 7 days, then 500 mg for 7 days. Patients who did not tolerate a dose-escalation (as evidenced by symptoms believed to be due to study drug) were returned to the last tolerated dose level. However, in such cases, dose escalation was re-attempted at least one additional time. The highest tolerable tirasemtiv dose for each individual patient was the dose that patient received for the remaining 9 weeks (i.e., the maximum tolerated dose [MTD] phase) of the study. Patients randomized to placebo underwent a corresponding dummy dose titration.

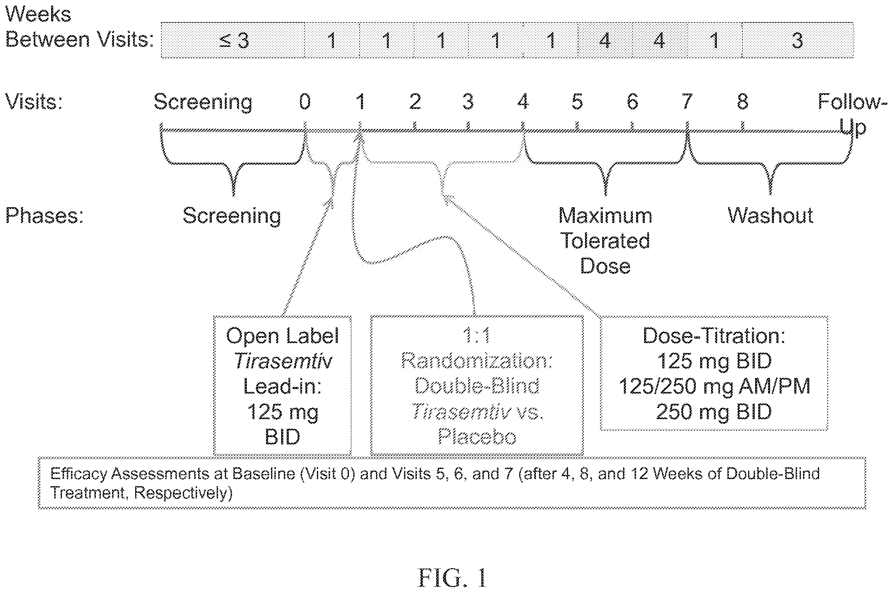

Randomization was stratified by riluzole use. Patients taking riluzole (which is metabolized by CYP 1A2, an enzyme inhibited by tirasemtiv) prior to study entry continued taking riluzole but at half the approved dose (i.e., 50 mg/day) because prior studies demonstrated that riluzole exposure approximately doubled when administered concomitantly with tirasemtiv. Clinical assessments occurred every 4 weeks during double-blind treatment; patients also returned for follow-up evaluations at 1 and 4 weeks after their final dose of double-blind study medication (**FIG. 1**).

The primary objective of BENEFIT-ALS was to assess the effect of tirasemtiv (administered BID up to a maximum daily dose of 500 mg) versus placebo on the total score of the revised form of the ALS Functional Rating Scale (ALSFRS-R). The primary endpoint was the change from baseline to the average of the ALSFRS-R total scores obtained at Visits 6 and 7 (i.e., after approximately 8 and 12 weeks of double-blind treatment). The primary analysis was conducted using all observed data during the randomized double-blind treatment period without imputation for missing data and based on the modified full analysis set (Modified FAS).

The secondary objectives included assessments of the effect of tirasemtiv versus placebo on measures of respiratory function (Maximum Voluntary Ventilation (MVV), Sniff Nasal Inspiratory Pressure (SNIP), SVC) and skeletal muscle function including handgrip fatigue and muscle-strength mega-score (i.e., the average z-score for muscle strength measurements across the following tested bilateral muscle groups: elbow flexion, wrist extension, knee extension, ankle dorsiflexion, and handgrip)). Secondary endpoints included the changes from baseline in each of the above mentioned measures (MVV, SNIP, SVC, and muscle-strength mega-score) for the Modified FAS, and the slope of each of the measures from baseline to Week 12 for the Modified FAS.

Eligible patients were males and females, 18 years of age and older, with a diagnosis of familial or sporadic ALS and meeting the World Federation of Neurology El Escorial criteria of definite ALS, probable ALS, laboratory-supported probable ALS, or possible ALS. Patients were to have an upright SVC > 50% of predicted for age, height, and sex, a diminished, but measurable, maximum voluntary grip strength in at least one hand (i.e., between 10-50 pounds for females and 10-70 pounds for males), and at least 4 of the 12 ALSFRS-R questions were to have been scored as a 2 or 3. Patients were to be able to swallow tablets without crushing, and in the opinion of the investigator, were expected to do so for the duration of the study. If the patient needed a caregiver, the caregiver was to be able to observe the patient's status. Clinical laboratory results were to be either within their respective normal ranges or if outside the normal range, deemed not clinically significant by the investigator. Patients taking riluzole were to have been on a stable dose for at least 30 days prior to screening. Patients not taking riluzole prior to study entry were not permitted to take riluzole at any dose during the course of the study.

A total of 711 patients were enrolled in the study and began treatment with open-label tirasemtiv 125 mg BID. After the one-week open-label phase, 302 patients were randomized to placebo and 303 to tirasemtiv. Of these randomized patients, 269 in the placebo group and 204 in the tirasemtiv group completed the study, while 33 on placebo and 99 on tirasemtiv prematurely discontinued study drug, primarily due to AEs (12 patients on placebo 78 patients on tirasemtiv) and patient withdrawal of consent (7 placebo, 12 tirasemtiv).

Safety data presented were based on the safety analysis set, defined as all patients who received at least 1 dose of double-blind study drug. Of the 605 randomized patients, 596 (295 placebo, 301 tirasemtiv) received at least 1 dose of double-blind study drug and were included in the safety analysis set. Data for the primary and secondary efficacy endpoints were analyzed with the Modified FAS. This analysis set included all patients who received at least 1 dose of double-blind study drug and had at least 1 efficacy assessment during the double-blind treatment period, and excluded 156 patients who were randomized in blocks containing patients affected by a study drug assignment error in which 58 patients randomized to and treated with double-blind tirasemtiv were dispensed double-blind placebo instead. Based on these criteria, the Modified FAS consisted of 388 patients (210 placebo, 178 tirasemtiv).

Key demographic and baseline characteristics are summarized for the Modified FAS in Table 1.

**Table 1**

| **Characteristic** | **Placebo (N=210)** | **Tirasemti v (N=178)** | **Overall (N=388)** |
|---|---|---|---|
| *Demographics and Physical Baseline Characteristics* | | | |
| Age (years), Mean (SD) | 56.8 (10.65) | 56.1 (11.74) | 56.5 (11.15) |
| Sex, Male, n (%) | 148 (70.5%) | 131 (73.6%) | 279 (71.9%) |
| Race, White/Caucasian, n (%) | 175 (83.3%) | 149 (83.7%) | 324 (83.5%) |
| Ethnicity, Not Hispanic or Latino, n(%) | 171 (81.4%) | 148 (83.1 %) | 319 (82.2%) |
| BMI (kg/m²), Mean (SD) | 26.80 (4.381) | 26.70 (4.426) | 26.76 (4.396) |

| *Disease History* | | | |
|---|---|---|---|
| Months since diagnosis, Mean(SD) | 12.1 (17.1) | 13.8 (20.8) | 12.9 (18.9) |
| Months since symptom onset, Mean (SD) | 26.7 (23.74) | 30.6 (32.07) | 28.5 (27.90) |

| *Baseline Disease Characteristics* | | | |
|---|---|---|---|
| ALSFRS-R Total Score, Mean (SD) | 37.3 (4.20) | 37.0 (4.70) | 37.2 (4.43) |
| Slow Vital Capacity, % predicted, Mean (SD)^{a} | 89.67 (17.184) | 85.66 (19.337) | 87.83 (18.290) |
| Maximum Voluntary Ventilation (L/min), Mean (SD) | 75.10 (35.681) | 72.62 (35.048) | 73.96 (35.368) |
| Sniff Nasal Inspiratory Pressure (cm H₂O), Mean (SD) | 61.4 (25.68) | 57.8 (25.09) | 59.7 (25.44) |
| Sub-maximum handgrip fatigue at 60% of Target in the Weaker Hand (sec), Mean (SD) | 84.48 (49.105) | 78.31 (50.356) | 81.67 (49.706) |

| | | | |
|---|---|---|---|
| ^{a} Baseline characteristics were not statistically different between treatment groups, except for SVC (p = 0.0125; obtained from an analysis of variance model, with treatment groups, riluzole use, and pooled site as fixed effects). | | | |

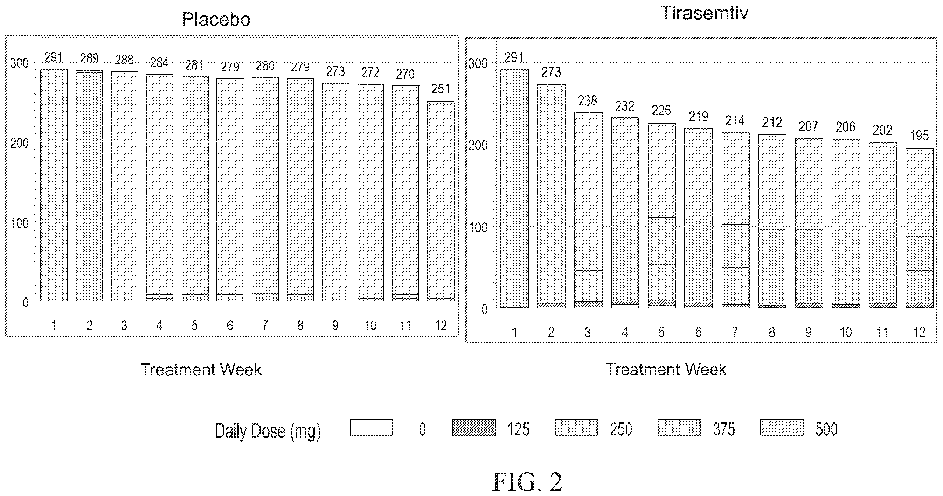

The doses of study drug (placebo or tirasemtiv) that patients received during each week of the double-blind phase of the study are depicted in **FIG 2****.**

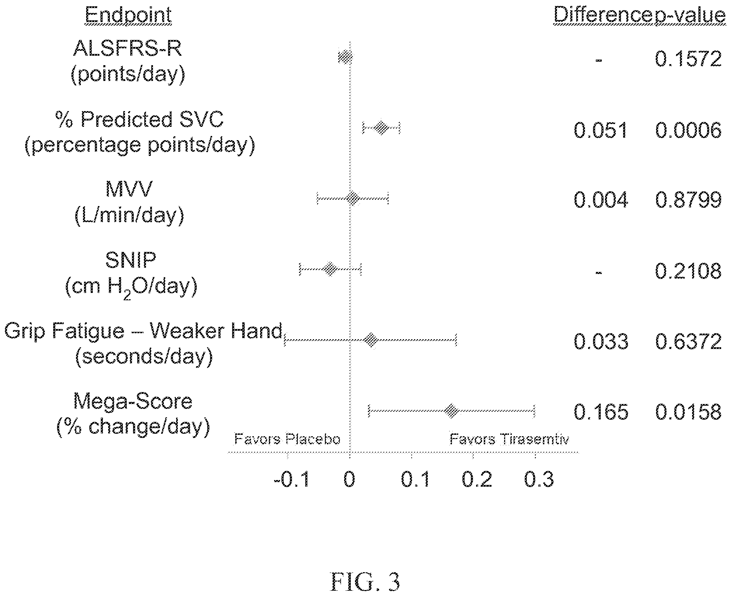

The results of the study are summarized in **FIG. 3**, which depicts the difference in the slope of change from baseline between placebo and tirasemtiv for the various endpoints. Within the Modified FAS, the changes from baseline to the average of ALSFRS total score at Visits 6 and 7 were not statistically different between treatment groups. The LS mean changes from baseline were -2.40 in the placebo group and -2.98 in the tirasemtiv group. The LS mean ± standard error (SE) difference between treatment groups (i.e., tirasemtiv response minus placebo response) was -0.58 ± 0.366 (95% confidence interval: -1.30, 0.14; p = 0.114).

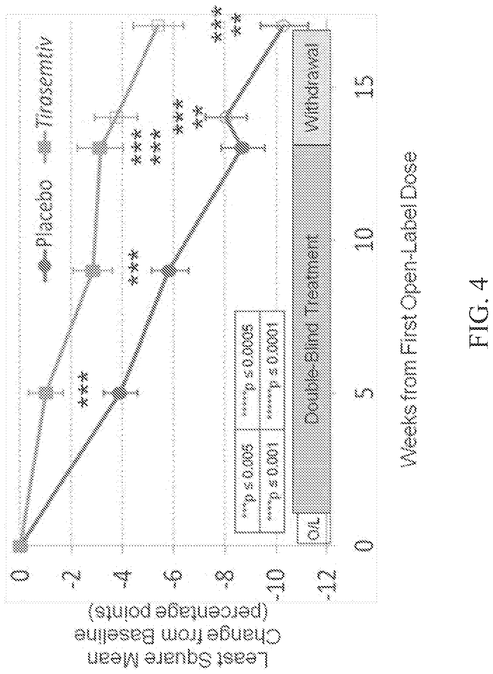

Treatment with tirasemtiv resulted in a statistically significant and potentially clinically meaningful reduction in the decline of SVC, a measure of strength of the skeletal muscles responsible for breathing. Changes from baseline in SVC and the slope of the changes from baseline to Week 12 are summarized in Table 2 and depicted in **FIG 4****.** In addition, Table 2 provides changes in SVC from baseline to post treatment time points of Weeks 13 and 16 (i.e., measured 1 and 4 weeks after the last dose of double-blind study drug).

**Table 2**

| **Endpoint** | | | **Placebo (N = 210)** | ***Tirasemtiv* (N = 178)** | **p-Value** |
|---|---|---|---|---|---|
| **SVC** | | | | | |
| Baseline % predicted, Mean (SD) | | | 89.67 (17.184) | 85.66 (19.337) | — |
| | Changes from baseline (LS mean [SE]) | | | | |
| | | Week 4 | ―3.89 (0.62) | ―0.99 (0.68) | 0.001 |
| | | Week 8 | ―5.81 (0.68) | ―2.85 (0.77) | 0.004 |
| | | Week 12 | ―8.66 (0.80) | ―3.12 (0.90) | < 0.0001 |
| | Slope | | ―0.0905 | ―0.0394 | — |
| | Difference in Slope | | | 0.051 | 0.0006 |
| Post-treatment Measurements | | | | | |
| Changes from baseline (LS mean [SE]) | | | | | |
| | | Week 13^{a} | -8.03 (0.77) | -3.75 (0.84) | 0.0002 |
| | | Week 16^{b} | -10.30 (0.90) | -5.39 (0.98) | 0.0002 |

| | | | | | |
|---|---|---|---|---|---|
| ^{a} 1 week after last dose of double-blind study drug ^{b} 4 weeks after last dose of double-blind study drug | | | | | |

At all measured time points during the double-blind phase of the study (Weeks 4, 8, and 12), patients in the tirasemtiv group had statistically significantly less decline in SVC than patients in the placebo group. After 12 weeks of treatment, the LS mean ± SE change from baseline in SVC was -8.66% ± 0.80% in the placebo group and ―3.12% ± 0.90% in the tirasemtiv group (p < 0.0001). The slopes of the changes from baseline to Week 12 were also statistically significantly different between treatment groups (p = 0.0006), with patients in the tirasemtiv group showing a slower rate of decline than those in the placebo group. The statistically significant difference between treatment groups in change from baseline in SVC persisted through at least 4 weeks after the last dose of study drug.

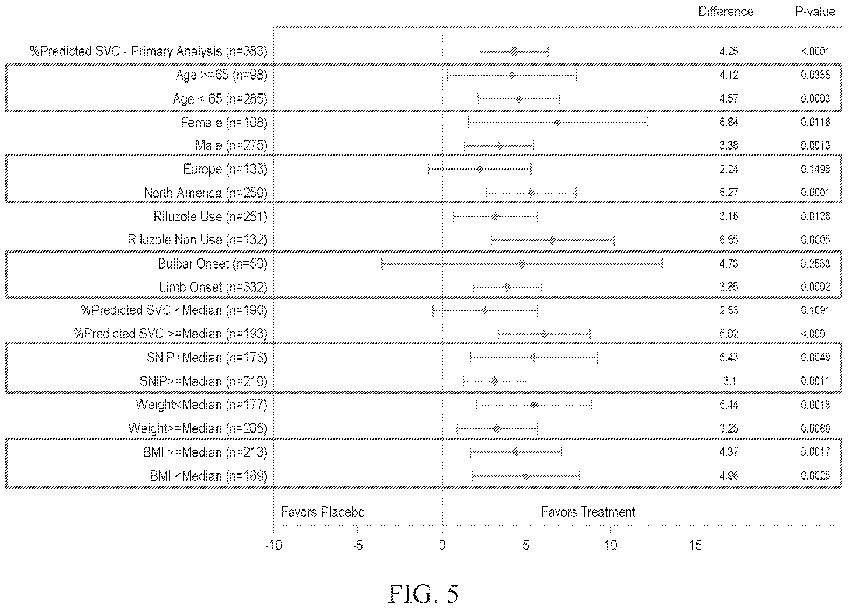

Subgroup analyses of change from baseline in SVC showed that tirasemtiv reduced the decline in SVC compared to placebo regardless of age, gender, riluzole use, or BMI (**FIG. 5**). Subgroups with the largest and most significant differences between treatment groups in change from baseline to average SVC after 8 and 12 weeks of double-blind treatment were as follows: females (with a treatment difference of 6.84%, p = 0.012); non-riluzole users (6.55%, p = 0.0005); and patients with baseline SVC ≥ median at baseline (6.02%, p< 0.0001).

Changes from baseline to Weeks 4, 8, and 12 in MVV, SNIP, and muscle strength mega-score, along with the slope of each of the measures from baseline to Week 12, are summarized in Table 3. There was no statistically significant difference between treatment groups for MVV and handgrip fatigue. The rate of decline for SNIP was not statistically significantly different between tirasemtiv and placebo (p = 0.21); however, the tirasemtiv group had a statistically significantly greater decrease in SNIP at Weeks 4 and 8 compared with the placebo group (p = 0.012 and 0.050, respectively). The muscle strength mega-score, a measure of strength based on the percent change from baseline across several muscle groups, declined more slowly for the tirasemtiv group than the placebo group (p = 0.0158 for the difference in slope); however, there were no statistical differences between treatment groups for mega-score at any of the measured time points.

**Table 3**

| **Endpoint** | | | **Placebo (N = 210)** | ***Tirasemtiv* (N = 178)** | **p-Value** |
|---|---|---|---|---|---|
| **MVV** | | | | | |
| | Baseline (L/min), Mean (SD) | | 75.10 (35.681) | 72.62 (35.048) | — |
| | Changes from baseline (LS mean [SE]) | | | | |
| | | Week 4 | ―2.30 (1.27) | ―2.89 (1.38) | 0.745 |
| | | Week 8 | ―2.90 (1.40) | ―2.14 (1.58) | 0.713 |
| | | Week 12 | ―5.64 (1.46) | ―5.45 (1.65) | 0.930 |
| | Slope | | ―0.061 | ―0.0567 | — |
| | Difference in Slope | | | 0.0044 | 0.8799 |

| **SNIP** | | | | | |
|---|---|---|---|---|---|
| Baseline (cm H₂O), Mean (SD) | | | 61.4 (25.68) | 57.8 (25.09) | — |
| | Changes from baseline (LS mean [SE]) | | | | |
| | | Week 4 | ―0.04 (1.18) | ―4.33 (1.28) | 0.012 |
| | | Week 8 | ―0.58 (1.09) | ―3.55 (1.23) | 0.066 |
| | | Week 12 | ―1.20 (1.31) | ―5.03 (1.48) | 0.050 |
| | Slope | | ―0.0218 | ―0.053 | — |
| | Difference in Slope | | | ―0.0312 | 0.2108 |

| **Handgrip Fatigue at 60% of Target in the Weaker Hand** | | | | | |
|---|---|---|---|---|---|
| Baseline (sec), Mean (SD) | | | 84.48 (49.105) | 78.31 (50.356) | — |
| | Changes from baseline (LS mean [SE]) | | | | |
| | | Week 4 | 1.89 (3.14) | ―1.77 (3.51) | 0.437 |
| | | Week 8 | 5.51 (3.03) | 4.60 (3.52) | 0.845 |
| | | Week 12 | ―1.98 (3.40) | ―0.57 (3.95) | 0.788 |
| | Slope | | 0.0126 | 0.0458 | — |
| | Difference in Slope | | | 0.0332 | 0.6372 |

| **Muscle Strength Mega-Score** | | | | | |
|---|---|---|---|---|---|
| Baseline (Mega-Score Units), Mean (SD) | | | ―0.06 (0.48) | ―0.06 (0.48) | — |
| | Changes from baseline (LS mean [SE]) | | | | |
| | | Week 4 | ―0.38 (1.74) | ―4.36 (1.90) | 0.546 |
| | | Week 8 | ―5.66 (2.27) | ―7.43 (2.63) | 0.610 |
| | | Week 12 | ―15.77 (2.56) | ―10.78 (2.98) | 0.205 |
| | Slope | | ―0.2751 | ―0.1104 | |
| | Difference in Slope | | | 0.1647 | 0.0158 |

An overview of treatment-emergent AEs during the double-blind phase of the study is provided in Table 4, and commonly reported AEs during the double-blind phase are summarized in Table 5. Serious AEs during the double-blind phase of the study were reported for 16 patients (5.4%) in the placebo group and 27 patients (9.0%) in the tirasemtiv group. By the Medical Dictionary for Regulatory Affairs (MedDRA) system organ class, the most commonly reported SAEs involved respiratory, thoracic and mediastinal disorders (2.0% placebo, 1.3% tirasemtiv). By MedDRA preferred term, AEs that were reported for more than 1 patient included the following: dysphagia (0.3% placebo, 0.7% tirasemtiv), pneumonia (0.3% placebo, 0.7% tirasemtiv), confusional state (0% placebo, 0.7% tirasemtiv), delirium (0% placebo, 0.7% tirasemtiv), and respiratory failure (1.0% placebo, 0.5% tirasemtiv).

During the first 4 weeks of the double-blind phase, 38.5% of patients (10/26) terminated placebo treatment while 73.2% (71/97) terminated tirasemtiv treatment. After the first 4 weeks of double- blind treatment, early termination rates for the two treatment groups were nearly parallel. Adverse events that most commonly led to early discontinuation were dizziness (0.3% placebo, 9.3% tirasemtiv), fatigue (0.3% placebo, 5.0% tirasemtiv), and confusional state (0% placebo, 5.0% tirasemtiv). Deaths during the double-blind phase of the study were reported for 3 patients (1.0%) in the placebo group and 2 patients (0.7%) in the tirasemtiv group. Deaths in the placebo group were attributed to hypercapnia, respiratory failure, and respiratory tract infection. Deaths in the tirasemtiv group were attributed to pneumonia and respiratory failure.

**Table 4**

| **No. (%) of patients with any treatment-emergent event ^{a, b}** | | **Placebo (N=295)** | ***Tirasemtiv* (N=301)** |
|---|---|---|---|
| AE | | 258 (87.5%) | 291 (96.5%) |
| | Grade 3 AE | 33 (11.2%) | 61 (20.3%) |
| | Grade 4 AE | 5 (1.7%) | 9 (3.0%) |
| | Grade 5 AE | 3 (1.0%) | 2 (0.7%) |
| SAE | | 16 (5.4%) | 27 (9.0%) |
| AE leading to early termination | | 14 (4.7%) | 78 (25.9%) |
| Deaths | | 3 (1.0%) | 2 (0.7%) |

| | | | |
|---|---|---|---|
| ^{a} Includes AEs that started during the open-label phase if they persisted for at least 96 hours after the first dose of double-blind study drug. ^{b} The severity of each AE was assessed by assigning a Grade of 1, 2, 3, 4, or 5 according to the National Cancer Institute Common Terminology Criteria for Adverse Events. | | | |

**Table 5**

| **MedDRA Preferred Term** | **Placebo (N=295)** | ***Tirasemtiv* (N=301)** |
|---|---|---|
| Any Adverse Event^{a} | 87.5% | 96.7% |
| Dizziness | 19.7% | 50.8% |
| Fatigue | 14.2% | 33.2% |
| Nausea | 7.8% | 21.9% |
| Headache | 11.2% | 17.9% |
| Asthenia | 12.5% | 15.9% |
| Muscle spasms | 5.4% | 15.0% |
| Muscular weakness | 6.8% | 11.3% |
| Somnolence | 3.7% | 13.0% |
| Contusion | 8.5% | 7.3% |
| Insomnia | 4.1% | 10.3% |
| Decreased appetite | 3.1% | 10.0% |
| Diarrhea | 5.8% | 7.3% |
| Nasopharyngitis | 6.4% | 6.3% |
| Respiratory failure | 5.8% | 6.3% |
| Confusional state | 1.0% | 11.0% |
| Constipation | 5.8% | 6.3% |

| | | |
|---|---|---|
| MedDRA = Medical Dictionary for Regulatory Affairs, version 15.1 ^{a} Includes AEs that started during the open-label phase and persisted for at least 96 hours after the first dose of double-blind study drug. | | |

During the double-blind phase of the study, patients in both treatment groups had a mean decrease in body weight. However, at each measured time point (Weeks 4, 8, and 12), patients in the tirasemtiv group had statistically significantly greater weight loss, compared with baseline values, than patients in the placebo group (Table 6).

**Table 6**

| **Weight (kg)** | **Placebo (N=210)** | ***Tirasemtiv* (N=178)** | **p-Value^{a}** |
|---|---|---|---|
| Baseline, Mean (SD) | 80.1 (15.10) | 80.4 (15.31) | — |
| Change from Baseline at Week 4, LS Mean (SE) | ―0.41 (0.130) | ―0.83 (0.142) | 0.0274 |
| Change from Baseline at Week | ―0.61 (0.164) | ―1.30 (0.181) | 0.0043 |
| 8, LS Mean (SE) | | | |
| Change from Baseline at Week 12, LS Mean (SE) | -0.79 (0.164) | -1.70 (0.244) | 0.0058 |

| | | | |
|---|---|---|---|
| LS mean = least square mean, SE = standard error ^{a} p-value from repeated measures model. | | | |

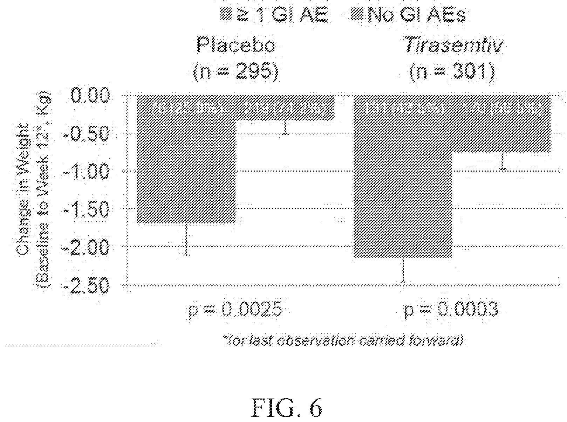

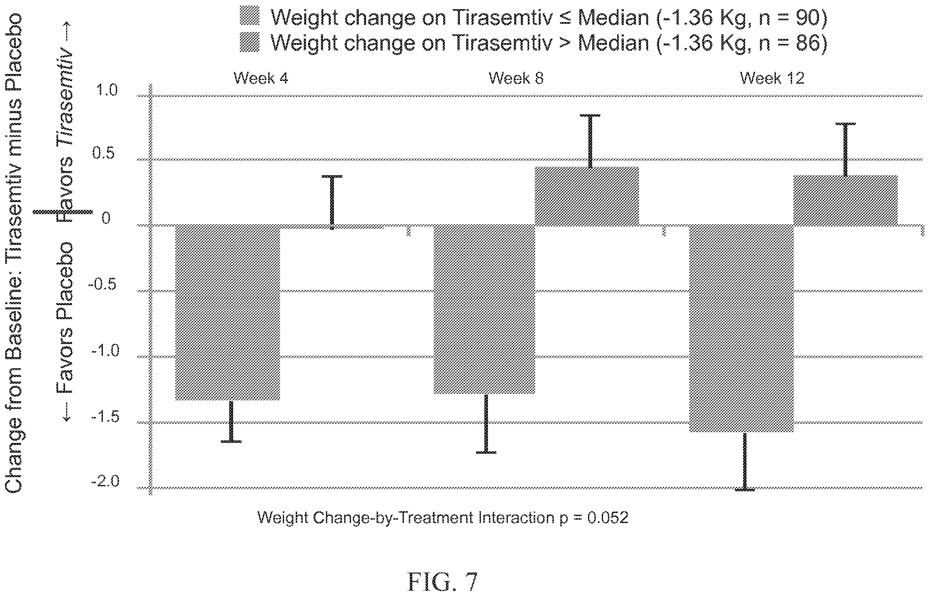

In this study, weight loss appeared to be associated with gastrointestinal (GI) AEs, which were reported for 76 patients (25.8%) in the placebo group and 131 patients (43.5%) in the tirasemtiv group. Patients in either treatment group who had at least 1 GI AE lost significantly more weight than patients who had not experienced a GI AE (**FIG. 6**). The decline ALSFRS-R score in the tirasemtiv group may be at least in part attributable to weight loss, as a small beneficial effect of tirasemtiv is suggested in the tirasemtiv patients who lost less weight (**FIG. 7**). However, weight loss may not entirely explain the ALSFRS-R results because while the difference in weight change between patients on tirasemtiv and those on placebo persisted through 4 weeks after the final dose of double-blind study drug, their ALSFRS-R total scores were nearly identical by 4 weeks after the final dose of double-blind study drug.

While some embodiments have been shown and described, the invention is defined in the appended claims. Accordingly, it is to be understood that the present invention has been described by way of illustration and not limitations on the scope of the claims.

## Claims

1. A skeletal muscle troponin activator for use in a method of reducing decline in slow vital capacity in a subject, the method comprising administering to the subject a therapeutically effective amount of the skeletal muscle troponin activator, wherein the skeletal muscle troponin activator is 1-(2-(((3-fluoro-1-(3-fluoropyridin-2-yl)cyclobutyl)methyl)amino)pyrimidin-5-yl)-1H-pyrrole-3-carboxamide, or a pharmaceutically acceptable salt thereof.

2. A skeletal muscle troponin activator for use in a method of reducing progressive respiratory decline in a subject with amyotrophic lateral sclerosis (ALS), the method comprising administering to the subject a therapeutically effective amount of the skeletal muscle troponin activator, wherein the skeletal muscle troponin activator is 1-(2-(((3-fluoro-1-(3-fluoropyridin-2-yl)cyclobutyl)methyl)amino)pyrimidin-5-yl)-1H-pyrrole-3-carboxamide, or a pharmaceutically acceptable salt thereof,
wherein the decline of the subject's slow vital capacity (SVC) is reduced.

3. The skeletal muscle troponin activator for use in a method of reducing decline in slow vital capacity in a subject according to claim 1, wherein the subject has a neurodegenerative disease.

4. The skeletal muscle troponin activator for use in a method of reducing decline in slow vital capacity in a subject according to claim 3, wherein the neurodegenerative disease is:
(i) selected from the group consisting of amyotrophic lateral sclerosis (ALS), myasthenia gravis, spinal muscle atrophy (SMA), and muscular dystrophy; or
(ii) amyotrophic lateral sclerosis (ALS).

5. The skeletal muscle troponin activator for use in a method of reducing decline in slow vital capacity in a subject according to any one of claims 1, 3, or 4, or for use in a method of reducing progressive respiratory decline in a subject with amyotrophic lateral sclerosis (ALS) according to claim 2, wherein the skeletal muscle troponin activator is administered orally.

6. The skeletal muscle troponin activator for use in a method of reducing decline in slow vital capacity in a subject according to any one of claims 1 and 3-5, or for use in a method of reducing progressive respiratory decline in a subject with amyotrophic lateral sclerosis (ALS) according to any one of claims 2 and 5, further comprising administration of a second therapeutic agent.

7. The skeletal muscle troponin activator for use in a method of reducing decline in slow vital capacity in a subject according to claim 6, or for use in a method of reducing progressive respiratory decline in a subject with amyotrophic lateral sclerosis (ALS) according to claim 6, wherein the second therapeutic agent is an amyotrophic lateral sclerosis (ALS) treatment.

8. The skeletal muscle troponin activator for use in a method of reducing decline in slow vital capacity in a subject according to claim 7, or for use in a method of reducing progressive respiratory decline in a subject with amyotrophic lateral sclerosis (ALS) according to claim 7, wherein the second therapeutic agent is riluzole.

9. The skeletal muscle troponin activator for use in a method of reducing decline in slow vital capacity in a subject according to any one of claims 1 and 3-8, or for use in a method of reducing progressive respiratory decline in a subject with amyotrophic lateral sclerosis (ALS) according to any one of claims 2, and 5-8, wherein the daily dose of 1-(2-(((3-fluoro-1-(3-fluoropyridin-2-yl)cyclobutyl)methyl)amino)pyrimidin-5-yl)-1H-pyrrole-3-carboxamide, or a pharmaceutically acceptable salt thereof, is from 100 to 1000 mg per day.

## Patentansprüche

1. Skelettmuskel-Troponinaktivator zur Verwendung bei einem Verfahren zur Reduzierung des Rückgangs der langsamen Vitalkapazität bei einem Individuum, wobei das Verfahren das Verabreichen einer therapeutisch wirksamen Menge des Skelettmuskel-Troponinaktivators an das Individuum umfasst, wobei es sich beim Skelettmuskel-Troponinaktivator um 1-(2-(((3-Fluor-1-(3-fluorpyridin-2-yl)cyclobutyl)methyl)amino)pyrimidin-5-yl)-1H-pyrrol-3-carboxamid oder ein pharmazeutisch annehmbares Salz davon handelt.

2. Skelettmuskel-Troponinaktivator zur Verwendung bei einem Verfahren zur Reduzierung einer progressiven respiratorischen Verschlechterung bei einem Individuum mit amyotrophischer Lateralsklerose (ALS), wobei das Verfahren das Verabreichen einer therapeutisch wirksamen Menge des Skelettmuskel-Troponinaktivators an das Individuum umfasst, wobei es sich beim Skelettmuskel-Troponinaktivator um 1-(2-(((3-Fluor-1-(3-fluorpyridin-2-yl)cyclobutyl)methyl)amino)pyrimidin-5-yl)-1H-pyrrol-3-carboxamid oder ein pharmazeutisch annehmbares Salz davon handelt,
wobei der Rückgang der langsamen Vitalkapazität (SVC) des Individuums reduziert wird.

3. Skelettmuskel-Troponinaktivator zur Verwendung bei einem Verfahren zur Reduzierung des Rückgangs der langsamen Vitalkapazität bei einem Individuum nach Anspruch 1, wobei das Individuum eine neurodegenerative Erkrankung hat.

4. Skelettmuskel-Troponinaktivator zur Verwendung bei einem Verfahren zur Reduzierung des Rückgangs der langsamen Vitalkapazität bei einem Individuum nach Anspruch 3, wobei die neurodegenerative Erkrankung:
(i) aus der Gruppe bestehend aus amyotrophischer Lateralsklerose (ALS), Myasthenia gravis, spinaler Muskelatrophie (SMA) und Muskeldystrophie ausgewählt oder
(ii) amyotrophische Lateralsklerose (ALS) ist.

5. Skelettmuskel-Troponinaktivator zur Verwendung bei einem Verfahren zur Reduzierung des Rückgangs der langsamen Vitalkapazität bei einem Individuum nach einem der Ansprüche 1, 3 oder 4 oder zur Verwendung bei einem Verfahren zur Reduzierung einer progressiven respiratorischen Verschlechterung bei einem Individuum mit amyotrophischer Lateralsklerose (ALS) nach Anspruch 2, wobei der Skelettmuskel-Troponinaktivator oral verabreicht wird.

6. Skelettmuskel-Troponinaktivator zur Verwendung bei einem Verfahren zur Reduzierung des Rückgangs der langsamen Vitalkapazität bei einem Individuum nach einem der Ansprüche 1 und 3-5 oder zur Verwendung bei einem Verfahren zur Reduzierung einer progressiven respiratorischen Verschlechterung bei einem Individuum mit amyotrophischer Lateralsklerose (ALS) nach einem der Ansprüche 2 und 5, das weiterhin die Verabreichung eines zweiten Therapeutikums umfasst.

7. Skelettmuskel-Troponinaktivator zur Verwendung bei einem Verfahren zur Reduzierung des Rückgangs der langsamen Vitalkapazität bei einem Individuum nach Anspruch 6 oder zur Verwendung bei einem Verfahren zur Reduzierung einer progressiven respiratorischen Verschlechterung bei einem Individuum mit amyotrophischer Lateralsklerose (ALS) nach Anspruch 6, wobei das zweite Therapeutikum eine Behandlung der amyotrophischen Lateralsklerose (ALS) ist.

8. Skelettmuskel-Troponinaktivator zur Verwendung bei einem Verfahren zur Reduzierung des Rückgangs der langsamen Vitalkapazität bei einem Individuum nach Anspruch 7 oder zur Verwendung bei einem Verfahren zur Reduzierung einer progressiven respiratorischen Verschlechterung bei einem Individuum mit amyotrophischer Lateralsklerose (ALS) nach Anspruch 7, wobei das zweite Therapeutikum Riluzol ist.

9. Skelettmuskel-Troponinaktivator zur Verwendung bei einem Verfahren zur Reduzierung des Rückgangs der langsamen Vitalkapazität bei einem Individuum nach einem der Ansprüche 1 und 3-8 oder zur Verwendung bei einem Verfahren zur Reduzierung einer progressiven respiratorischen Verschlechterung bei einem Individuum mit amyotrophischer Lateralsklerose (ALS) nach einem der Ansprüche 2 und 5-8, wobei die tägliche Dosis von 1-(2-(((3-Fluor-1-(3-fluorpyridin-2-yl)cyclobutyl)methyl)amino)pyrimidin-5-yl)-1H-pyrrol-3-carboxamid oder eines pharmazeutisch annehmbaren Salzes davon 100 bis 1000 mg pro Tag beträgt.

## Revendications

1. Activateur de troponine de muscle du squelette pour une utilisation dans un procédé de réduction du déclin de la capacité vitale lente chez un sujet, le procédé comprenant l'administration au sujet d'une quantité thérapeutiquement efficace de l'activateur de troponine de muscle du squelette, l'activateur de troponine de muscle du squelette étant le 1-(2-(((3-fluoro-1-(3-fluoropyridin-2-yl)cyclobutyl)méthyl)amino)pyrimidin-5-yl)-1H-pyrrole-3-carboxamide, ou un sel pharmaceutiquement acceptable correspondant.

2. Activateur de troponine de muscle du squelette pour une utilisation dans un procédé de réduction du déclin respiratoire progressif chez un sujet atteint d'une sclérose latérale amyotrophique (SLA), le procédé comprenant l'administration au sujet d'une quantité thérapeutiquement efficace de l'activateur de troponine de muscle du squelette, l'activateur de troponine de muscle du squelette étant le 1-(2-(((3-fluoro-1-(3-fluoropyridin-2-yl)cyclobutyl)méthyl)amino)pyrimidin-5-yl)-1H-pyrrole-3-carboxamide, ou un sel pharmaceutiquement acceptable correspondant,
le déclin de la capacité vitale lente (CVL) du sujet étant réduit.

3. Activateur de troponine de muscle du squelette pour une utilisation dans un procédé de réduction du déclin de la capacité vitale lente chez un sujet selon la revendication 1, le sujet étant atteint d'une maladie neurodégénérative.

4. Activateur de troponine de muscle du squelette pour une utilisation dans un procédé de réduction du déclin de la capacité vitale lente chez un sujet selon la revendication 3, la maladie neurodégénérative étant :
(i) choisie dans le groupe constitué par la sclérose latérale amyotrophique (SLA), la myasthénie grave, l'atrophie musculaire spinale (AMS), et la dystrophie musculaire ; ou
(ii) la sclérose latérale amyotrophique (SLA).

5. Activateur de troponine de muscle du squelette pour une utilisation dans un procédé de réduction du déclin de la capacité vitale lente chez un sujet selon l'une quelconque des revendications 1, 3 et 4, ou pour une utilisation dans un procédé de réduction du déclin respiratoire progressif chez un sujet atteint d'une sclérose latérale amyotrophique (SLA) selon la revendication 2, l'activateur de troponine de muscle du squelette étant administré oralement.

6. Activateur de troponine de muscle du squelette pour une utilisation dans un procédé de réduction du déclin de la capacité vitale lente chez un sujet selon l'une quelconque des revendications 1 et 3 à 5, ou pour une utilisation dans un procédé de réduction du déclin respiratoire progressif chez un sujet atteint d'une sclérose latérale amyotrophique (SLA) selon l'une quelconque des revendications 2 et 5, comprenant en outre l'administration d'un deuxième agent thérapeutique.

7. Activateur de troponine de muscle du squelette pour une utilisation dans un procédé de réduction du déclin de la capacité vitale lente chez un sujet selon la revendication 6, ou pour une utilisation dans un procédé de réduction du déclin respiratoire progressif chez un sujet atteint d'une sclérose latérale amyotrophique (SLA) selon la revendication 6, le deuxième agent thérapeutique étant un traitement de la sclérose latérale amyotrophique (SLA).

8. Activateur de troponine de muscle du squelette pour une utilisation dans un procédé de réduction du déclin de la capacité vitale lente chez un sujet selon la revendication 7, ou pour une utilisation dans un procédé de réduction du déclin respiratoire progressif chez un sujet atteint d'une sclérose latérale amyotrophique (SLA) selon la revendication 7, le deuxième agent thérapeutique étant le riluzole.

9. Activateur de troponine de muscle du squelette pour une utilisation dans un procédé de réduction du déclin de la capacité vitale lente chez un sujet selon l'une quelconque des revendications 1 et 3 à 8, ou pour une utilisation dans un procédé de réduction du déclin respiratoire progressif chez un sujet atteint d'une sclérose latérale amyotrophique (SLA) selon l'une quelconque des revendications 2, et 5 à 8, la dose quotidienne de 1-(2-(((3-fluoro-1-(3-fluoropyridin-2-yl)cyclobutyl)méthyl)amino)pyrimidin-5-yl)-1H-pyrrole-3-carboxamide, ou d'un sel pharmaceutiquement acceptable correspondant, étant de 100 à 1 000 mg par jour.
